(19) Europäisches Patentamt
European Patent Office
Office européen des brevets

(11) EP 1 113 802 B1

(12) EUROPEAN PATENT SPECIFICATION

(45) Date of publication and mention
of the grant of the patent:
30.06.2004 Bulletin 2004/27

(51) Int Cl.$^7$: A61K 31/513, A61K 31/52, A61P 31/20

(21) Application number: 99947376.2

(22) Date of filing: 17.09.1999

(86) International application number:
PCT/EP1999/006886

(87) International publication number:
WO 2000/016755 (30.03.2000 Gazette 2000/13)

(54) **ANTIVIRAL COMBINATIONS OF LAMIVUDINE AND ADEFOVIR**

ANTIVIRALE KOMBINATIONEN VON LAMIVUDIN UND ADEFOVIR

COMBINAISONS ANTIVIRALES DE LAMIVUDINE ET D'ADEFOVIR

(84) Designated Contracting States:
**AT BE CH CY DE DK ES FI FR GB GR IE IT LI LU MC NL PT SE**
Designated Extension States:
**SI**

(30) Priority: 18.09.1998 GB 9820420

(43) Date of publication of application:
11.07.2001 Bulletin 2001/28

(73) Proprietor: GLAXO GROUP LIMITED
Greenford, Middlesex UB6 ONN (GB)

(72) Inventors:
• BROWN, Nathaniel, A. Glaxo Wellcome Inc.
Research Triangle Park, NC 27709 (US)
• CONDREAY, Lynn, D. Glaxo Wellcome Inc.
Research Triangle Park, NC 27709 (US)
• GRAY, Douglas, Fraser Glaxo Wellcome plc
Greenford Middlesex UB6 0HE (GB)
• RUBIN, Marc Glaxo Wellcome Inc.
Research Triangle Park, NC 27709 (US)

(74) Representative: Hockley, Siân Catherine et al
GlaxoSmithKline
Corporate Intellectual Property (CN9.25.1)
980 Great West Road
Brentford, Middlesex TW8 9GS (GB)

(56) References cited:
WO-A-99/66936

• BARTNOF H. S.: "Preveon shows benefits for patients co-infected with HIV and HBV" HIV AND HEPATITIS.COM, [Online] 18 August 1999 (1999-08-18), XP002132867 Retrieved from the Internet: <URL:http://www.hivandhepatitis.com/hiv/v1 0089904.html> [retrieved on 2000-03-13]
• PERRILLO: "Gilead Presents Preliminary Clinical Data demostrating activity of adefovir dipivoxil against lamivudine-resistant Hepatitis B virus" GILEAD SCIENCES PRESS RELEASE ARCHIVE, [Online] 9 April 1999 (1999-04-09), XP002132868 Retrieved from the Internet: <URL:http://www.gilead.com/webpage_templ at es/frame_home.php3> [retrieved on 2000-03-13]
• THOMPSON M. ET AL: "Randomized Study of Adefovir Dipivoxil (ADV) in combination with Indinavir (IDV) and reverse transcriptase inhibitors for treatment-naive HIV infected patients" ABSTRACTS AND POSTERS IAPAC, [Online] 8 November 1998 (1998-11-08), XP002132869 Retrieved from the Internet: <URL:http://www.iapac.org/conferences/glas gow98/gileadglasgow5.html> [retrieved on 2000-03-13]

EP 1 113 802 B1

- ONO-NITA, S. K. (1) ET AL: "Susceptibility of lamivudine resistant hepatitis B virus to other antivirals: Adefovir and lobucavir." HEPATOLOGY, (OCT., 1998) VOL. 28, NO. 4 PART 2, PP. 165A. MEETING INFO.: BIENNIAL SCIENTIFIC MEETING OF THE INTERNATIONAL ASSOCIATION FOR THE STUDY OF THE LIVER AND THE 49TH ANNUAL MEETING AND POSTGRADUATE COURSES OF THE AMERICAN ASSOCIATION FOR THE , XP000890075
- MULATO, A.S. ET AL: "Anti-HIV activity of adefovir (PMEA) and PMPA in combination with antiretroviral compounds: in vitro analyses" ANTIVIRAL RES. (1997), 36(2), 91-97 , XP000890091
- SHAW, T. ET AL: "Synergistic inhibition of in vitro hepadnaviral replication by PMEA and penciclovir or lamivudine." ANTIVIRAL RESEARCH, (1997) VOL. 34, NO. 2, PP. A51. MEETING INFO.: MEETING OF THE INTERNATIONAL SOCIETY FOR ANTIVIRAL RESEARCH AND THE TENTH INTERNATIONAL CONFERENCE ON ANTIVIRAL RESEARCH ATLANTA, GEORGIA, USA APRIL 6-11, 1997 , XP000890096
- DE CLERCQ E: "Perspectives for the treatment of hepatitis B virus infections." INTERNATIONAL JOURNAL OF ANTIMICROBIAL AGENTS, (1999 JUL) 12 (2) 81-95. REF: 72 , XP000890077
- PESSOA M.G. ET AL: "Update on clinical trials in the treatment of hepatitis B." JOURNAL OF GASTROENTEROLOGY AND HEPATOLOGY, (1999) 14/SUPPL. (S6-S11). , XP000890090
- PETERS M G ET AL: "Fulminant hepatic failure resulting from lamivudine -resistant hepatitis B virus in a renal transplant recipient: durable response after orthotopic liver transplantation on adefovir dipivoxil and hepatitis B immune globulin." TRANSPLANTATION, (1999 DEC 27) 68 (12) 1912-4. , XP000890081

## Description

The present invention relates to therapeutic combinations comprising (2R,cis)-4-amino-1-(2-hydroxymethyl-1,3-oxathiolan-5-yl)-pyrimidin-2-one (lamivudine) and a second therapeutic agent bis(pivaloyloxymethyl)(9-[(R)-2-(phosphonomethoxy)ethyl]adenine [the oral prodrug of PMEA ((9-[(R)-2-(phosphonomethoxy)ethyl]adenine or adefovir), adefovir dipivoxil]. The present invention is also concerned with pharmaceutical compositions containing combinations comprising (2R,cis)-4-amino-1-(2-hydroxymethyl-1,3-oxathiolan-5-yl)-pyrimidin-2-one and a second therapeutic agent selected from (9-[(R)-2-(phosphonomethoxy)ethyl]adenine and bis(pivaloyloxymethyl)(9-[(R)-2-(phosphonomethoxy)ethyl]adenine and their use for the manufacture of a medicament for the treatment of HBV infections including infections with HBV mutants bearing resistance to nucleoside and/or non-nucleoside inhibitors of the replication of the hepatitis B virus.

Hepatitis B is a viral disease transmitted orally or parentally by contaminated material such as blood or blood products, contaminated needles, sexually, and vertically from infected or carrier mothers to their off-spring. In those areas of the world where the disease is common, vertical transmission at an early age results in a high proportion of infected individuals becoming chronic carriers of hepatitis B. An estimated 350 million people world-wide are chronically infected with hepatitis B and as many as 150 million may die from liver disease in the absence of intervention.

Currently, the only established approach to treatment of hepatitis B is repeated injections of interferon, which may be associated with unpleasant side effects, and produces a long lasting response in only one third or less of those treated. Interferon is an immune modulator designed to boost the disease fighting ability of the immune system.

Lamivudine has been reported to be effective against HBV in a two year study, showing that most patients showed substantially reduced levels of viral replication with 52% maintaining undetectable levels of virus thorough to the end of the second year.

Adefovir has been reported to possess anti-HBV activity in vitro, and the oral prodrug of adefovir, adefovir dipivoxil, has been shown to be active against HBV replication in vivo and is currently in phase II clinical studies with patients who have chronic hepatitis B viral infection.

There has been a report that there is lack of cross-resistance to PMEA for Human hepatitis B DNA polymerase which expresses lamivudine codons, *X. Xiong et al.(Hepatology Vol 26, No. 4, Pt. 2, 1997, Abstract No. 1211).*

*Mulato et al.* (*Antiviral Research, 36, 1997, 91-97*) describe the anti-HIV activity of PMEA in combination with antiretroviral compounds such as d4T, ddC, AZT, ddl, lamivudine (3TC), nelfinavir, ritonavir, indinavir and saquinavir. The combination of PMEA with lamivudine was found to exhibit additive inhibition of HIV replication.

The use of combinations of the invention may give rise to equivalent antiviral effect with reduced toxicity, or an increase in drug efficacy because synergy between compounds occurs. Lower overall drug doses will also possibly reduce the frequency of occurrence of drug resistant variants of HBV.

We have now found that lamivudine exhibits unexpected advantages when used in combination with adefovir. In particular the combinations shows a statistically significant synergistic anti-HBV effect. Early results have shown that the combination of lamivudine and adefovir dipivoxil also exhibits a synergistic anti-HBV-effect. It is a feature of this invention that the use of these drug combinations will provide synergistic antiviral effects, more complete viral suppression, viral suppression over longer periods, limit the emergence of drug resistant HBV mutants and allow better management of drug related toxicites. The use of these drug combinations may also result in a decrease of the number of, for example, tablets administered a day, therefore may increase patient compliance.

As will be appreciated by those skilled in the art, references herein to treatment extend to prophylaxis as well as to the treatment of established infections and symptoms.

Pharmaceutically acceptable salts of lamivudine, adefovir, or adefovir dipivoxil include those derived from pharmaceutically acceptable inorganic and organic acids. Examples of suitable acids include hydrochloric, hydrobromic, sulphuric, nitric, perchloric, fumaric, maleic, phosphoric, glycollic, lactic, salicylic, succinic, toluene- p-sulphonic, tartaric, acetic, citric, methanesulphonic, formic, benzoic, malonic, naphthalene-2-sulphonic and benzenesulphonic acids. Other acids such as oxalic acid, while not in themselves pharmaceutically acceptable may be useful in the preparation of salts useful as intermediates in obtaining compounds of the invention and their pharmaceutically acceptable acid addition salts.

Salts derived from appropriate bases include alkali metal (e.g. sodium), alkaline earth metal (e.g. magnesium), ammonium and $NR_{4+}$ (where R is $C_{1-4}$ alkyl) salts.

Preferred esters of lamivudine, adefovir or adefovir dipivoxil are independently selected from the following group: (1) carboxylic acid esters in which the non-carbonyl moiety of the carboxylic acid portion of the ester grouping is selected from straight or branched chain alkyl (for example, methyl, n-propyl, t-butyl, or n-butyl), cycloalkyl, alkoxyalkyl (for example, methoxymethyl), aralkyl (for example, benzyl), aryloxyalkyl (for example, phenoxymethyl), aryl (for example, phenyl optionally substituted by, for example, halogen, $C_{1-4}$ alkyl, or $C_{1-4}$ alkoxy), or amino; (2) sulphonate esters, such as alkyl- or aralkylsulphonyl (for example, methanesulphonyl); (3) amino acid esters (for example, L-valyl or L-isoleucyl); and (4) phosphonate esters. In such esters, unless otherwise specified, any alkyl moiety present advantageously contains from 1 to 18 carbon atoms, particularly from 1 to 6 carbon atoms, more particularly from 1 to 4 carbon atoms. Any cycloalkyl moiety present in such esters advantageously contains from 3 to 6 carbon atoms. Any aryl moiety present in such esters advantageously comprises a phenyl group. Any reference to any of the above compounds also includes a reference to a physiologically acceptable salt thereof.

Particularly preferred esters are the mono-, di-, and triphosphate esters of lamivudine (which may be optionally blocked), or any other compound which upon administration to a human subject is capable of providing (directly or indirectly) said mono-, di-, or triphosphate ester.

Thus according to one aspect, the present invention provides a combination comprising (2R,cis)-4-amino-1-(2-hydroxymethyl-1,3-oxathiolan-5-yl)-pyrimidin-2-one or a pharmaceutically acceptable derivative thereof and a second therapeutic agent bis(pivaloyloxymethyl)(9-[(R)-2-(phosphonomethoxy)ethyl]adenine or a pharmaceutically acceptable derivative thereof, wherein (2R,cis)-4-amino-1-(2-hydroxymethyl-1,3-oxathiolan-5-yl)-pyrimidin-2-one and the second therapeutic agent are present in the range 40:1 to 1:1 by weight.

Combinations as described above may herein after be referred to as combinations according to the invention.

As used herein "pharmaceutically acceptable derivative" includes any pharmaceutically acceptable salt, ester or salt of such ester, of lamivudine, adefovir or adefovir dipivoxil or any other compound which, upon administration to the recipient, is capable of providing (directly or indirectly) such a compound or an antivirally active metabolite or residue thereof.

The present invention further provides combinations according to the invention for use in therapy, particularly in the treatment of an HBV infection including infections resistant to nucleoside and/or non-nucleoside inhibitors of the replication of the hepatitis B virus.

According to another aspect, the present invention provides the use of a combination comprising (2R,cis)-4-amino-1-(2-hydroxymethyl-1,3-oxathiolan-5-yl)-pyrimidin-2-one or a pharmaceutically acceptable derivative thereof and a second therapeutic agent selected from (9-[(R)-2-(phosphonomethoxy)ethyl]adenine or a pharmaceutically acceptable derivative thereof, and bis(pivaloyloxymethyl)(9-[(R)-2-(phosphonomethoxy)ethyl]adenine or a pharmaceutically acceptable derivative thereof, wherein (2R,cis)-4-amino-1-(2-hydroxymethyl-1,3-oxathiolan-5-yl)-pyrimidin-2-one and the second therapeutic agent are present in the range 40:1 to 1:1 by weight, for the manufacture of a medicament for the treatment of an HBV infection.

It will be appreciated that the compounds of the combination may be administered simultaneously, either in the same or different pharmaceutical composition, or sequentially. If there is sequential administration, the delay in administering the second active ingredient should not be such as to lose the benefit of a synergistic therapeutic effect of the combination of the active ingredients. It will also be understood that lamivudine, and adefovir dipivoxil, or the pharmaceutically acceptable derivatives thereof or adefovir or the pharmaceutically acceptable derivatives thereof, whether presented simultaneously or sequentially, may be administered individually or in any combination thereof. Lamivudine, and adefovir dipivoxil or adefovir are preferably administered simultaneously or sequentially in separate pharmaceutical formulations, most preferably simultaneously.

Preferably the combination according to the invention is administered as a single combined formulation.

The present invention also provides the use of lamivudine for the manufacture of a medicament for administration simultaneously or sequentially with adefovir or adefovir dipivoxil for the treatment of HBV infections. It will be appreciated that lamivudine, adefovir dipivoxil, or adefovir or any combination thereof (excluding adefovir and adefovir dipivoxil), may be used in the manufacture of the above medicament.

According to the invention the lamivudine and adefovir dipivoxil or adefovir are present in a synergistic ratio.

The synergistic effects of the combination of lamivudine and adefovir dipivoxil or adefovir or pharmaceutically acceptable derivatives thereof are seen over a ratio of 40:1 to 1:1 (by weight), preferably 25:1 to 15: 1 (by weight).

Conveniently each compound will be employed in the combination in an amount at which it exhibits anti-HBV activity when used alone.

The amount of a combination of lamivudine, adefovir or adefovir dipivoxil required to be effective as an anti-HBV agent will, of course, vary and is ultimately at the discretion of the medical practitioner. The factors to be considered include the route of administration and nature of the formulation, the animal's body weight, age and general condition and the nature and severity of the disease to be treated.

in general for lamivudine a suitable daily dose will be in the range of from about 0.1 to about 50 mg per kilogram body weight of the recipient per day, preferably in the range of 0.5 to 20 mg per kilogram body weight per day, most preferably in the range of 0.5 to 2 mg per kilogram body weight per day.

The compound is conveniently administered at a level of about 100 mg per day.

For adefovir dipivoxil a suitable daily dose will be in the range of from about 0.01 to about 10 mg per kilogram body weight of the recipient per day, preferably in the range of 0.01 to 1 mg per kilogram body weight per day, most preferably in the range of 0.01 to 0.05 mg per kilogram body weight per day.

Conveniently adefovir dipivoxil is administered at a level of about 10 mg per day.

For adefovir, a suitable daily dose will be in the range of from about 0.01 to about 10 mg per kilogram body weight of the recipient per day, preferably in the range of 0.01 to 1 mg per kilogram body weight per day, most preferably in the range of 0.01 to 0.05 mg per kilogram body weight per day.

Conveniently adefovir is administered at a level of about 10 mg per day.

Unless otherwise indicated all weights of active ingredients are calculated in terms of the drug per se. In the case of a pharmaceutically acceptable derivatives of lamivudine, adefovir dipivoxil or adefovir, or a solvate of any thereof the figures would be increased proportionately. The desired dose is preferably presented as two, three, four, five, six or more sub-doses administered at appropriate intervals throughout the day. These sub-doses may be administered in unit dosage forms, for example, containing from 1 to 1500 mg, preferably from 5 to 1000 mg, most preferably from 5 to 500 mg of active ingredient per unit dosage form. Alternatively, if the condition of the recipient so requires, the dose may be administered as a continuous infusion.

The components of the combination which may be referred to as active ingredients may be administered for therapy to an animal e.g. a mammal including a human in a conventional manner.

While it is possible for the active ingredients of the combination to be administered as the raw chemical it is preferable to present them as a pharmaceutical composition. Pharmaceutical compositions according to the present invention comprise a combination according to the invention in association with one or more pharmaceutically acceptable carriers or excipients and optionally other therapeutic agents. The carrier(s) must be acceptable in the sense of being compatible with the other ingredients of the formula and not deleterious to the recipient thereof. When the individual components of the combination are administered separately they are generally each presented as a pharmaceutical composition. The references hereinafter to compositions refer unless otherwise stated to compositions containing either the combination or a component thereof.

According to another aspect the present invention provides a pharmaceutical formulation for use in the treatment of HBV comprising (2R,cis)-4-amino-1-(2-hydroxymethyl-1,3-oxathiolan-5-yl)-pyrimidin-2-one or a pharmaceutically acceptable derivative thereof and a second therapeutic agent selected from (9-[(R)-2-(phosphonomethoxy)ethyl] adenine or a pharmaceutically acceptable derivative thereof and bis(pivaloyloxymethyl)(9-[(R)-2-(phosphonomethoxy) ethyl]adenine or a pharmaceutically acceptable derivative thereof, wherein (2R,cis)-4-amino-1-(2-hydroxymethyl-1,3-oxathiolan-5-yl)-pyrimidin-2-one and the second therapeutic agent are present in the range 40:1 to 1:1 by weight.

A combination of lamivudine and adefovir dipivoxil or adefovir or pharmaceutically acceptable derivatives thereof may conveniently be presented as a pharmaceutical composition with one or more pharmaceutically acceptable carrier thereof in a unitary dosage form. A convenient unitary dosage formulation contains the active ingredients in amounts of from 1 mg to 2 g each, for example, 2 mg to 200 mg such as 25 to 150 mg of lamivudine and 5 to 60 mg of adefovir or adefovir dipivoxil.

Pharmaceutical compositions may also be prescribed to the patient in "patient packs" containing the whole course of treatment in a single package, usually a blister pack. Patient packs have an advantage over traditional prescriptions, where a pharmacists divides a patients supply of a pharmaceutical from a bulk supply, in that the patient always has access to the package insert contained in the patient pack, normally missing in traditional prescriptions. The inclusion of a package insert has been shown to improve patient compliance with the physicians instructions.

It will be understood that the administration of the combination of the invention by means of a single patient pack, or patients packs of each composition, within a package insert diverting the patient to the correct use of the invention is a desirable additional feature of this invention.

The patient pack may comprise at least one active ingredient of the combination according to the invention and an information insert containing directions on the use of the combination of the invention.

The patient pack may also comprise a double pack comprising in association for separate administration lamivudine and adefovir dipivoxil or adefovir or pharmaceutically acceptable derivatives thereof.

Compositions include those suitable for oral, rectal, nasal, topical (including transdermal, buccal and sublingual), vaginal or parenteral (including subcutaneous, intramuscular, intravenous and intradermal) administration. The compositions may conveniently be presented in unit dosage form and may be prepared by any methods well known in the art of pharmacy. Such methods represent a further feature of the present invention and include the step of bringing into association the active ingredients with the carrier which constitutes one or more accessory ingredients. In general, the formulations are prepared by uniformly and intimately bringing into association the active ingredients with liquid carriers or finely divided solid carriers or both, and then if necessary shaping the product.

Compositions of the present invention suitable for oral administration may be presented as discrete units such as capsules, caplets, cachets or tablets each containing a predetermined amount of the active ingredients; as a powder or granules; as a solution or a suspension in an aqueous or non-aqueous liquid; or as an oil-in-water liquid emulsion or a water-in-oil liquid emulsion. The active ingredient may also be presented as a bolus, electuary or paste.

A tablet may be made by compression or moulding, optionally with one or more accessory ingredients. Compressed tablets may be prepared by compressing in a suitable machine the active ingredients in a free-flowing form such as a powder or granules, optionally mixed with a binder (e.g. povidone, gelatin, hydroxypropylmethyl cellulose), lubricant, inert diluent, preservative, disintegrant (e.g. sodium starch glycollate, cross-linked povidone, cross-linked sodium carboxymethyl cellulose) surface-active or dispersing agent. Moulded tablets may be made by molding a mixture of the powdered compound moistened with an inert liquid diluent in a suitable machine. The tablets may optionally be coated or scored and may be formulated so as to provide slow or controlled release of the active ingredients therein using, for example, hydroxypropylmethyl cellulose in varying proportions to provide the desired release profile. Tablets may optionally be provided with an enteric coating, to provide release in parts of the gut other than the stomach.

Preferably the combinations according to the invention are administered orally.

Compositions suitable for topical administration in the mouth include lozenges comprising the active ingredients in a flavoured base, usually sucrose and acacia or tragacanth; pastilles comprising the active ingredient in an inert basis such as gelatin and glycerin, or sucrose and acacia; and mouthwashes comprising the active ingredient in a suitable liquid carrier. Compositions for rectal administration may be presented as a suppository with a suitable base comprising, for example, cocoa butter or a salicylate.

Topical administration may also be by means of a transdermal iontophoretic device.

Formulations suitable for vaginal administration may be presented as pessaries, tampons, creams, gels, pastes, foams or spray formulations containing in addition to the active ingredient such carriers as are known in the art to be appropriate.

Pharmaceutical formulations suitable for rectal administration wherein the carrier is a solid are most preferably presented as unit dose suppositories. Suitable carriers include cocoa butter and other materials commonly used in the art. The suppositories may be conveniently formed by admixture of the active combination with the softened or melted carrier(s) followed by chilling and shaping in moulds.

Formulations suitable for parenteral administration include aqueous and nonaqueous isotonic sterile injection solutions which may contain anti-oxidants, buffers, bacteriostats and solutes which render the formulation isotonic with the blood of the intended recipient; and aqueous and non-aqueous sterile suspensions which may include suspending agents and thickening agents; and liposomes or other microparticulate systems which are designed to target the compound to blood components or one or more organs. The formulations may be presented in unit-dose or multi-dose sealed containers, for example, ampoules and vials, and may be stored in a freeze-dried (lyophilized) condition requiting only the addition of the sterile liquid carrier, for example water for injection, immediately prior to use. Extemporaneous injection solutions and suspensions may be prepared from sterile powders, granules and tablets of the kind previously described.

Preferred unit dosage formulations are those containing a daily dose or daily subdose of the active ingredients, as herein before recited, or an appropriate fraction thereof.

It should be understood that in addition to the ingredients particularly mentioned above the formulations of this invention may include other agents conventional in the art having regard to the type of formulation in question, for example, those suitable for oral administration may include such further agents as sweeteners, thickeners and flavouring agents.

The compounds of the combination of the present invention may be obtained in a conventional manner.

Methods for the preparation of lamivudine are described in International Patent Applications Numbers. WO91/17159, and WO 95/29174.

Methods for the preparation of adefovir are described in European Patent No. 206459.

Methods for the preparation of adefovir dipivoxil are described in European Patent No. 481214.

The following examples are intended for illustration only and are not intended to limit the scope of the invention in any way. "Active ingredient" denotes lamivudine, adefovir dipivoxil or adefovir or multiples thereof or a physiologically functional derivative of any of the aforementioned compounds.

Example 1: Tablet Formulation

The following formulations A, B and C are prepared by wet granulation of the ingredients with a solution of povidone, followed by addition of magnesium stearate and compression.

| Formulation A | |
| --- | --- |
|  | mg/tablet |
| Active Ingredient A | 100 |
| Active Ingredient B | 30 |
| Lactose B.P. | 105 |
| Povidone B.P. | 7 |
| Sodium Starch Glycollate | 10 |

(continued)

| Formulation A | |
|---|---|
| | mg/tablet |
| Magnesium Stearate | 3 |
| | $\overline{255}$ |

| Formulation B | |
|---|---|
| | mg/tablet |
| Active Ingredient A | 100 |
| Active Ingredient B | 30 |
| Lactose B.P. | 75 |
| Avicel PH 101 | 30 |
| Povidone B.P. | 7 |
| Sodium Starch Glycollate | 10 |
| Magnesium Stearate | 3 |
| | $\overline{255}$ |

| Formulation C | |
|---|---|
| | mg/tablet |
| Active Ingredient A | 100 |
| Active Ingredient B | 5 |
| Lactose B.P. | 100 |
| Starch | 25 |
| Povidone | 2 |
| Magnesium Stearate | 2 |
| | $\overline{234}$ |

The following formulations, D and E, are prepared by direct compression of the admixed ingredients. The lactose in formulation E is of the direct compression type (Dairy Crest - "Zeparox").

| Formulation D | |
|---|---|
| | mg/tablet |
| Active Ingredient A | 100 |
| Active Ingredient B | 30 |
| Pregelatinized Starch NF15 | 75 |
| | $\overline{205}$ |

| Formulation E | |
|---|---|
| | mg/tablet |
| Active Ingredient A | 100 |
| Active Ingredient B | 5 |
| Lactose B.P. | 70 |
| Avicel | 50 |
| | $\overline{225}$ |

Formulation F (Controlled Release Formulation)

The formulation is prepared by wet granulation of the ingredients with a solution of povidone followed by the addition of magnesium stearate and compression.

|  | mg/tablet |
|---|---|
| Active Ingredient A | 100 |
| Active Ingredient B | 30 |
| Hydroxypropylmethylcellulose (Methocel K4M Premium) | 28 |
| Lactose B.P. | 13 |
| Povidone B.P. | 7 |
| Magnesium Stearate | 2 |
|  | 180 |

Drug release takes place over a period of about 6-8 hours and is complete after 12 hours.

Example 2: Capsule Formulations

Formulation A

A capsule formulation is prepared by admixing the ingredients of formulation D in Example 1 above and filling into a two-part hard gelatin capsule. Formulation B (infra) is prepared in a similar manner.

| Formulation B | |
|---|---|
|  | mg/capsule |
| Active Ingredient A | 100 |
| Active Ingredient B | 5 |
| Lactose B.P. | 70 |
| Sodium Starch Glycollate | 10 |
| Magnesium Stearate | 1 |
|  | 186 |

| Formulation C | |
|---|---|
|  | mg/capsule |
| Active Ingredient A | 100 |
| Active Ingredient B | 30 |
| Macrogel 4000 B.P. | 170 |
|  | 300 |

Capsules of formulation C are prepared by melting the Macrogel 4000 B.P., dispersing the active ingredient in the melt and filling the melt into a two-part hard gelatin capsule.

| Formulation D | |
|---|---|
|  | mg/capsule |
| Active Ingredient A | 100 |
| Active Ingredient B | 5 |
| Lecithin | 50 |
| Arachis Oil | 50 |
|  | 205 |

Capsules of formulation D are prepared by dispersing the active ingredient in the lecithin and arachis oil and fitting the dispersion into soft, elastic gelatin capsules.

Formulation E (Controlled Release Capsule)

The following controlled release capsule formulation is prepared by extruding ingredients a, b, and c using an extruder, followed by spheronization of the extrudate and drying. The dried pellets are then coated with release-controlling membrane (d) and filled into a two-piece, hard gelatin capsule.

|     |                          | mg/capsule |
| --- | ------------------------ | ---------- |
| (a) | Active Ingredient A      | 100        |
|     | Active Ingredient B      | 30         |
| (b) | Microcrystalline Cellulose | 60       |
| (c) | Lactose B.P.             | 60         |
| (d) | Ethyl Cellulose          | 6          |
|     |                          | 256        |

Example 3: Injectable Formulation

| Formulation A | |
| --- | --- |
|     | mg |
| Active Ingredient A | 100 |
| Active Ingredient B | 5 |
| Hydrochloric Acid Solution 0.1 M or | |
| Sodium Hydroxide Solution 0.1 M q.s. to pH | 4.0 to 7.0 |
| Sterile water q.s. to | 10 ml |

The active ingredient is dissolved in most of the water (35°-40°C) and the pH adjusted to between 4.0 and 7.0 with the hydrochloric acid or the sodium hydroxide as appropriate. The batch is then made up to volume with the water and filtered through a sterile micropore filter into a sterile 10 ml amber glass vial (type 1) and sealed with sterile closures and overseals.

| Formulation B | |
| --- | --- |
| Active Ingredient A | 125 mg |
| Sterile, Pyrogen-free, pH 7 Phosphate Buffer, q. s. to | 25 ml |

Example 4: Intramuscular injection

| Active Ingredient A | 100 mg |
| --- | --- |
| Active Ingredient B | 30 mg |
| Benzyl Alcohol | 0.067 g |
| Glycofurol 75 | 0.94 g |
| Water for injection q.s. to | 3.00 ml |

The active ingredient is dissolved in the glycofurol. The benzyl alcohol is then added and dissolved, and water added to 3 ml. The mixture is then filtered through a sterile micropore filter and sealed in sterile 3 ml amber glass vials (type 1).

Example 5: Syrup

| Active Ingredient A | 100 mg |
|---|---|
| Active Ingredient B | 5 mg |
| Sorbitol Solution | 0.6 g |
| Glycerol | 0.85 g |
| Sodium Benzoate | 0.0025 g |
| Flavour, Peach 17.42.3169 | 0.0125 ml |
| Purified Water q.s. to | 5.00 ml |

The active ingredient is dissolved in a mixture of the glycerol and most of the purified water. An aqueous solution of the sodium benzoate is then added to the solution, followed by addition of the sorbital solution and finally the flavour. The volume is made up with purified water and mixed well.

Example 6: Suppository

| | mg/capsule suppository |
|---|---|
| Active Ingredient A | 100 |
| Active Ingredient B | 30 |
| Hard Fat, B.P. (Witepsol H15 - Dynamit Nobel) | 1770 |
| | $\overline{1900}$ |

One-fifth of the Witepsol H15 is melted in a steam-jacketed pan at 45°C maximum. The active ingredient is sifted through a 200μM sieve and added to the molten base with mixing, using a Silverson fitted with a cutting head, until a smooth dispersion is achieved. Maintaining the mixture at 45°C, the remaining Witepsol H15 is added to the suspension and stirred to ensure a homogenous mix. The entire suspension is passed through a 250μm stainless steel screen and, with continuous stirring, is allowed to cool to 40°C. At a temperature of 38° C to 40°C, 2.02 g of the mixture is filled into suitable, 2 ml plastic moulds. The suppositories are allowed to cool to room temperature.

Example 7: Pessaries

| | mg/pessary |
|---|---|
| Active Ingredient A | 100 |
| Active Ingredient B | 5 |
| Anhydrate Dextrose | 160 |
| Potato Starch | 150 |
| Magnesium Stearate | 3 |
| | $\overline{418}$ |

The above ingredients are mixed directly and pessaries prepared by direct compression of the resulting mixture.

Biological Data

**Example 8.**

The human hepatobtastoma cell line (Hep-G2-2.2.15) which constitutively produces infectious HBV was seeded into 96 well microtiter plates at a density of $5 \times 10^3$ cells per well. These cells were treated with a combination of lamivudine and PMEA on triplicate plates. Culture media containing drugs was replenished every other day for 9 days, at which time supernatants were collected and analyzed for HBV content.

The lamivudine/PMEA combination was tested twice in triplicate in matrix fashion. Experiment 1 utilised a lamivudine range of 100 nM to 0.14 nM (3-fold dilutions in columns), and PMEA, 9-[(R)-2-(phosphonomethoxy)ethyl]adenine (adefovir), at concentrations of 1 μM to 10 nM (3.16 fold dilutions in rows).

Experiment 2 was performed with dilutions of Lamivudine ranging from 100nM to .045 nM (in 3-fold dilutions in columns), and a PMEA range of 5 μM to 0.16 nM (3.16 fold dilutions in rows). Both drugs were diluted in a separate 96 well microtiter plate, and subsequently transferred onto plates containing the cell monolayers. Cells are grown in 150 μl RPMI 1640 supplemented with 2 mM L-Glutamine and 10% fetal bovine serum. Prior to transfer of drug, 120 μl of media was removed from the cells, leaving 30 μl on the monolayers to prevent drying. 90 μl of fresh media without drug was added, followed by the addition of 30 μl of 5X drug dilutions. Lamivudine and PMEA were each tested on their respective plates individually at the same concentrations. Data were normalised to values obtained with non-drug treated cells, and expressed as a percent of control for analysis.

The method used for detection of HBV has been previously described *(Jansen RW, Johnson LC, Averett, DR. High-Capacity in vitro assessment* of *anti-hepatitis B virus compound selectivity by a virion-specific polymerase chain reaction assay. Antimicrob Agents Chem 1993; 37 (3): 441-447.)*. Briefly, HBV detection was performed by "capturing" virus from supernatants on Anti-HBsAg coated plates, washing, denaturing to release HBV DNA, performing PCR with biotinylated primers, streptavidin capture of biotinylated PCR products with concomitant probe hybridization, addition of substrate, and reading optical densities of the colorimetric reaction. Dilutions of a standardized HBV-containing supernatant were included on every plate, and HBV DNA concentrations of test wells were calculated from this HBV standard curve. The useful range of detection is at least .045 to 45 fg of HBV DNA, where 30 copies of the genome can be reliably detected. Samples were tested in conjunction with both positive (.448 fg/ul plasmid DNA) and negative (RPMI medium supplemented with 2 mM L-Glutamine and 10% Fetal calf serum) controls.

The average IC50 and standard error of the IC50s for the triplicate plates were calculated using SAS nonlinear regression to fit data to the Hill equation for each concentration response curve. When only a single determination of an IC50 for a particular dose combination could be made, the average of the standard errors from adjacent concentrations was used to estimate the standard error.

Fractional inhibitory concentrations (FIC50) were calculated for each combination and plotted using the isobologram representation *(Berenbaum, M.C. (1985) The Expected Effect ofa Combination of Agents: the General Solution. J. Theor. Biol. 114, 413-431)*. To assess statistical significance of synergy or antagonism, an unpaired t-test was used to compare each sum of paired FIC50 values with the theoretical value of 1. P values less than 0.05 were considered statistically significant. Comparison of P values between experiments must be interpreted with great care, as the experiments utilized different test concentration ranges (or ranges useable by the isobologram method). In some cases not all concentrations tested could support calculation of an IC50, since response was inhibited to a greater extent than 50 percent of control for all doses.

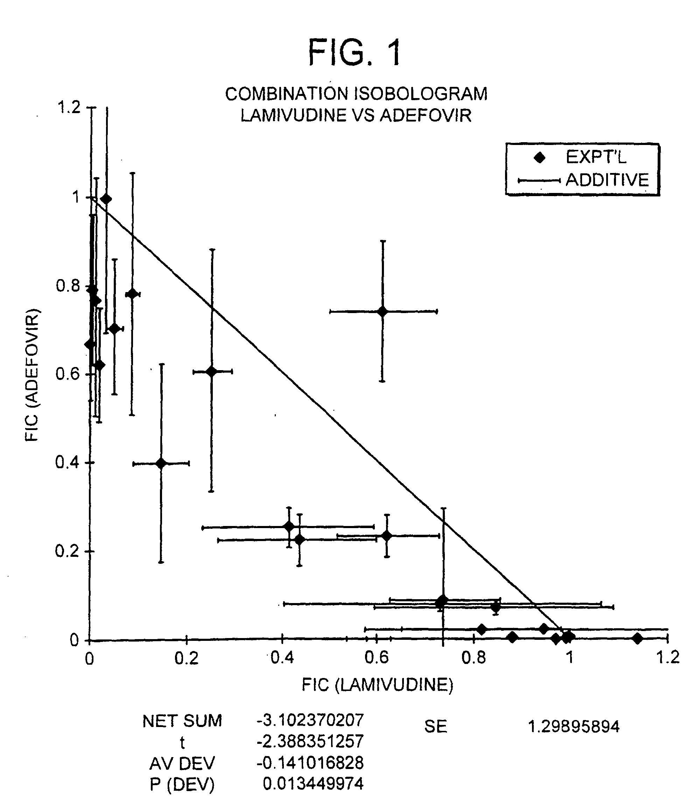

Figure 1 shows a single isobologram, produced by combining the data from both experiments, showing a statistically significant synergism.

## Example 9

The IC$_{50}$ for PMEA was determined against wild type (WT) and lamivudine resistant HBV transiently expressed in a cell culture system as described below. HepG2 cells were transiently transfected with plasmid containing the HBV genome that had the wild type sequence or contained the following lamivudine resistant mutations in the reverse transcriptase gene; M552I, M552V, L528M, L528MM552V. It was previously determined that only the M552I and L528MM552V mutations were observed in HBV infected patients that developed resistance to lamivudine therapy although the individual M552V and L528M mutations partially contributed to the loss in sensitivity of lamivudine against HBV replication *in vitro* (*Allen MI, Deslauriers M, Andrews CW, Tipples GA*, *Walters KA, Tyrrell DLJ, Brown N for the Lamivudine Clinical Investigation Group, and Condreay LD. Identification and characterization of mutations in hepatitis B virus resistant to lamivudine. HEPATOLOGY 1998; 27:9670-1677*). HepG2 cells were seeded into 96-well Costar plates at 6300 cells per well in 150 μl of HepG2 media (Dulbecco's Modified Eagle Medium (DMEM), containing 10% fetal bovine serum) and were incubated overnight at 37°C. For each transfected well, 75 ng of plasmid DNA and 0.5 μl of lipofectamine (Gibco) were incubated together for 30 minutes at room temperature in 12.5 μl of OptiMem (Gibco) prior to addition of DNA/lipofectamine mixture to cells. Each well was rinsed with 150 μl of unsupplemented DMEM. The DNA/lipofectamine mixture was added to each well in a total volume of 150 μl of OptiMem media. The cells were incubated with the DNA/lipofectamine solution for 5 hours at 37°C. After incubation, 150 μl of DMEM containing 20% serum was added to each well and plates were incubated overnight at 37°C. The media was replaced with 150 μl of HepG2 media only or media containing PMEA.

2.2.15 cells were seeded into 96-well Costar plates at 2250 cells per well in 150 μl of complete media (RPMI media containing 10% fetal bovine serum) and were incubated overnight at 37°C. The media was replaced with 150 µl of complete media only or media containing the desired concentration of PMEA.

Transfected cells, as well as 2.2.15 stable HBV-producer cells, were treated with control drug-free media or media containing PMEA every other day (day 1, 3, and 5). Final concentrations of PMEA for cell treatment were used at 25, 5, 1, 0.2, 0.04 µM (for WT plasmid transfected cultures and control 2.2.15 cells) or 125, 25, 5, 1, 0.2 µM (for all mutant plasmid transfected cultures).

HBV DNA levels were quantitated from media harvested from cells on day 7 using the methods described in *Jansen RW, Johnson LC, Averett, DR. High-Capacity in vitro assessment of anti-hepatitis B virus compound selectivity by a virion-specific polymerase chain reaction assay. Antimicrob Agents Chem 1993; 37 (3): 441-447*. Further details are given in Example 8.

Cytotoxicity due to drug treatment was determined using the DNA stain Bisbenzimide (H33342 3HCl 4H$_2$O; Calbiochem Company, La Jolla CA). After the media was harvested, the cells were fixed with 70% ethanol for 30 minutes. Cells were rinsed once with serum-free media and incubated with the DNA stain Bisbenzimide (H33342 3HCl 4H2O; Calbiochem Corporation, La Jolla CA) at 33 µg/ml for 1 hour at 37°C in serum-free medium. Fluorescent values per well were determined with a Millipore Cytofluor 2350 fluorescent plate reader (excitation, 355 nm; emission, 460 nm; arbitrary units). CC$_{50}$ values of each compound (concentration of compound that is cytotoxic for 50% of cells) were determined from the percent toxicity of each concentration of compound compared to untreated (no drug) cells using the method described below. Concentration response curves generated from each construct in the transient transfection experiment were fit to the Hill equation ($y = Vmax * (1 - (x^n/(k^n + x^n)))$) using non-linear regression to estimate the IC$_{50}$ (concentration of drug which inhibited HBV DNA production by 50%, compared to parallel drug-free cultures) and CC$_{50}$ of PMEA. The calculated IC$_{50}$ for each construct is shown with the 95% confidence bounds for the geometric means for n replicates. The program JMP (SAS, Cary NC) was used to perform student t-test evaluation of the data for determining statistically significant differences between treatment groups. The IC$_{50}$ of PMEA obtained against WT HBV produced by the 2.2.15 cells (0.43 uM) was also comparable to the IC$_{50}$ value (0.7 uM) using the same cell line. (*Heijtink RA, De Wilde GA, Kruining J, Berk L, Balzarini J, De Clercq E, Holy A, Schalam SW. Inhibitory Effect of 9-(phosphonylmethoxyethyl)-adenine(PMEA) on Human and Duck Hepatitis B Virus Infection. Antiviral Research 1993; 21 (2): 141-153*).

Table 1.

| Comparison of IC$_{50}$s of PMEA between WT HBV and HBV containing lamivudine resistant associated mutations *in vitro*. | | |
|---|---|---|
| HBV construct | IC$_{50}$ PMEA (95%CI) | x-fold change in IC$_{50}$ |
| 2.2.15 | 0.43 uM (0.3 - 0.7 uM) | - |
| WT | 0.53 uM (0.34 - 0.8 uM) | - |
| L528M/ M552V | 1.3 uM (0.4 - 4.3 uM)[+] | 2.5 |
| M552I | 7.5 uM (1.8 - 32 uM)* | 14 |
| M552V | 9.8 uM (3.1 - 31 uM)*[+] | 18 |
| L528M | 0.87 uM (0.4 - 2.0 uM) | 1.6 |

* Statistically different IC$_{50}$s of compound between mutant HBV and WT HBV, p < 0.05.

[+] IC$_{50}$s are statistically different from each other, p < 0.05.

For HepG2 cells, the cytotoxic IC$_{50}$ for PMPA was greater than 125 uM, the cytotoxic IC$_{50}$ for PMEA varied between 25 to 40 uM.

**Claims**

1. A combination comprising (2R,cis)-4-amino-1-(2-hydroxymethyl-1,3-oxathiolan-5-yl)-pyrimidin-2-one or a pharmaceutically acceptable derivative thereof and a second therapeutic agent bis(pivaloyloxymethyl)(9-[(R)-2-(phosphonomethoxy)ethyl]adenine or a pharmaceutically acceptable derivative thereof, wherein (2R,cis)-4-amino-1-(2-hydroxyrnethyl-1,3-oxathiolan-5-yl)-pyrimidin-2-one and the second therapeutic agent are present in the range 40:1 to 1:1 by weight.

2. A combination according to claim 1 wherein the ratio is in the range 25:1 to 15:1 by weight of active ingredients.

3. A combination according to claim 1 or 2 for use in medicine.

4. A pharmaceutical formulation comprising a combination according to any one of claims 1 to 3 in association with one or more pharmaceutically acceptable carriers therefor.

5. A pharmaceutical formulation for use in the treatment of HBV comprising (2R,cis)-4-amino-1-(2-hydroxymethyl-1,3-oxathiolan-5-yl)-pyrimidin-2-one or a pharmaceutically acceptable derivative thereof and a second therapeutic agent selected from (9-[(R)-2-(phosphonomethoxy)ethyl]adenine or a pharmaceutically acceptable derivative thereof and bis(pivaloyloxymethyl)(9-[(R)-2-(phosphonomethoxy)ethyl]adenine or a pharmaceutically acceptable derivative thereof, wherein (2R,cis)-4-amino-1-(2-hydroxymethyl-1,3-oxathiolan-5-yl)-pyrimidin-2-one and the second therapeutic agent are present in the range 40:1 to 1:1 by weight.

6. A formulation according to claims 4 or 5 in unit dosage form.

7. A formulation according to any one of claims 4 to 6 suitable for oral administration.

8. A formulation according to any one of claims 5 to 7 comprising between 25 to 150 mg of (2R,cis)-4-amino-1-(2-hydroxymethyl-1,3-oxathiolan-5-yl)-pyrimidin-2-one and 5 to 60 mg of bis(pivaloyloxymethyl)(9-[(R)-2-(phosphonomethoxy)ethyl]adenine.

9. A formulation according to claim 8 comprising 100 mg of (2R,cis)-4-amino-1-(2-hydroxymethyl-1,3-oxathiolan-5-yl)-pyrimidin-2-one and 10 mg of bis(pivaloyloxymethyl)(9-[(R)-2-(phosphonomethoxy)ethyl]adenine.

10. Use of a combination comprising (2R,cis)-4-amino-1-(2-hydroxymethyl-1,3-oxathiolan-5-yl)-pyrimidin-2-one or a pharmaceutically acceptable derivative thereof and a second therapeutic agent selected from (9-[(R)-2-(phosphonomethoxy)ethyl]adenine or a pharmaceutically acceptable derivative thereof and bis(pivaloyloxymethyl)(9-[(R)-2-(phosphonomethoxy)ethyl]adenine or a pharmaceutically acceptable derivative thereof, wherein (2R,cis)-4-amino-1-(2-hydroxymethyl-1,3-oxathiolan-5-yl)-pyrimidin-2-one and the second therapeutic agent are present in the range 40:1 to 1:1 by weight, for the manufacture of a medicament for the treatment of an HBV infection.

11. Use as claimed in claim 10 wherein the combination is as claimed in claim 1 or 2.

12. Use as claimed in claim 10 or 11 for the treatment of an HBV infection resistant to nucleoside and/or non-nucleoside inhibitors of the replication of the hepatitis B virus.

13. Use of (2R,cis)-4-amino-1-(2-hydroxymethyl-1,3-oxathiolan-5-yl)-pyrimidin-2-one or a pharmaceutically acceptable derivative thereof for the manufacture of a medicament for administration either simultaneously or sequentially with (9-[(R)-2-(phosphonomethoxy)ethyl]adenine or a pharmaceutically acceptable derivative thereof or bis(pivaloyloxymethyl)(9-[2-(phosphonomethoxy)ethyl]adenine or a pharmaceutically acceptable derivative thereof, wherein (2R,cis)-4-amino-1-(2-hydroxymethyl-1,3-oxathiolan-5-yl)-pyrimidin-2-one and (9-[(R)-2-(phosphonomethoxy)ethyl]adenine or bis(pivaloyloxymethyl)(9-[2-(phosphonomethoxy)ethyl]adenine are present in the range 40:1 to 1:1 by weight, for the treatment of an HBV infection.

14. Use of (9-[(R)-2-(phosphonomethoxy)ethyl]adenine or a pharmaceutically acceptable derivative thereof or bis (pivaloyloxymethyl)(9-[2-(phosphonomethoxy)ethyl]adenine or a pharmaceutically acceptable derivative thereof for the manufacture of a medicament for administration either simultaneously or sequentially with (2R,cis)-4-amino-1-(2-hydroxymethyl-1,3-oxathiolan-5-yl)-(1H)-pyrimidin-2-one or a pharmaceutically acceptable derivative thereof, wherein (9-[(R)-2-(phosphonomethoxy)ethyl]adenine or bis(pivaloyloxymethyl)(9-[2-(phosphonomethoxy)ethyl]adenine and (2R,cis)-4-amino-1-(2-hydroxymethyl-1,3-oxathiolan-5-yl)-pyrimidin-2-one are present in the range 1:40 to 1:1 by weight, for the treatment of an HBV infection.

15. A product comprising (2R,cis)-4-amino-1-(2-hydroxymethyl-1,3-oxathiolan-5-yl)-pyrimidin-2-one or a pharmaceutically acceptable derivative thereof as a preparation for simultaneous, separate or sequential use together with bis(pivaloyloxymethyl)(9-[2-(phosphonomethoxy)ethyl]adenine or a pharmaceutically acceptable derivative thereof in a ratio of 40:1 to 1:1 by weight in the treatment of an HBV infection.

16. A product comprising bis(pivaloyloxymethyl)(9-[2-(phosphonomethoxy)ethyl]adenine or a pharmaceutically acceptable derivative thereof as a preparation for simultaneous, separate or sequential use together with (2R,cis)-4-amino-1-(2-hydroxymethyl-1,3-oxathiolan-5-yl)-pyrimidin-2-one or a pharmaceutically acceptable derivative thereof in a ratio of 1:40 to 1:1 by weight in the treatment of an HBV infection.

17. A product comprising (2R,cis)-4-amino-1-(2-hydroxymethyl-1,3-oxathiolan-5-yl)-pyrimidin-2-one or a pharmaceutically acceptable derivative thereof and bis(pivaloyloxymethyl)(9-[2-(phosphonomethoxy)ethyl]adenine or a pharmaceutically acceptable derivative thereof in a ratio of 40:1 to 1:1 by weight as a combined preparation for simultaneous, separate or sequential use in the treatment of an HBV infection.

**Patentansprüche**

1. Kombination, die (2R,cis)-4-Amino-1-(2-hydroxymethyl-1,3-oxathiolan-5-yl)-pyrimidin-2-on oder ein pharmazeutisch akzeptables Derivat davon und ein zweites Therapeutikum, Bis(pivaloyloxymethyl)(9-[(R)-2-(phosphonomethoxy)ethyl]adenin oder ein pharmazeutisch akzeptables Derivat davon, umfaßt, worin (2R,cis)-4-Amino-1-(2-hydroxymethyl-1,3-oxathiolan-5-yl)-pyrimidin-2-on und das zweite Therapeutikum im Gewichtsbereich von 40:1 bis 1:1 vorhanden sind.

2. Kombination gemäß Anspruch 1, worin das Gewichtsverhältnis der aktiven Bestandteile im Bereich von 25:1 bis 15:1 ist.

3. Kombination gemäß Anspruch 1 oder 2 zur Verwendung in der Medizin.

4. Pharmazeutische Formulierung, die eine Kombination gemäß einem der Ansprüche 1 bis 3 in Verbindung mit einem oder mehreren pharmazeutisch akzeptablen Trägern dafür umfaßt.

5. Pharmazeutische Formulierung zur Verwendung in der Behandlung von HBV, umfassend (2R,cis)-4-Amino-1-(2-hydroxymethyl-1,3-oxathiolan-5-yl)-pyrimidin-2-on oder ein pharmazeutisch akzeptables Derivat davon und ein zweites Therapeutikum, das aus (9-[(R)-2-(Phosphonomethoxy)ethyl]adenin oder einem pharmazeutisch akzeptablen Derivat davon und Bis(pivaloyloxymethyl)-(9-[(R)-2-(phosphonomethoxy)ethyl]adenin oder einem pharmazeutisch akzeptablen Derivat davon ausgewählt ist, worin (2R,cis)-4-Amino-1-(2-hydroxymethyl-1,3-oxathiolan-5-yl)-pyrimidin-2-on und das zweite Therapeutikum im Gewichtsbereich von 40:1 bis 1:1 vorhanden sind.

6. Formulierung gemäß Anspruch 4 oder 5 in Einheitsarzneiform.

7. Formulierung gemäß einem der Ansprüche 4 bis 6, die zur oralen Verabreichung geeignet ist.

8. Formulierung gemäß einem der Ansprüche 5 bis 7, die 25 bis 150 mg (2R,cis)-4-Amino-1-(2-hydroxymethyl-1,3-oxathiolan-5-yl)-pyrimidin-2-on und 5 bis 60 mg Bis(pivaloyloxymethyl)(9-[(R)-2-(phosphonomethoxy)ethyl]adenin umfaßt.

9. Formulierung gemäß Anspruch 8, die 100 mg (2R,cis)-4-Amino-1-(2-hydroxymethyl-1,3-oxathiolan-5-yl)-pyrimidin-2-on und 10 mg Bis(pivaloyloxymethyl)(9-[(R)-2-(phosphonomethoxy)ethyl]adenin umfaßt.

10. Verwendung einer Kombination, die (2R,cis)-4-Amino-1-(2-hydroxymethyl-1,3-oxathiolan-5-yl)-pyrimidin-2-on oder ein pharmazeutisch akzeptables Derivat davon und ein zweites Therapeutikum, das aus (9-[(R)-2-(Phosphonomethoxy)ethyl]adenin oder einem pharmazeutisch akzeptablen Derivat davon und Bis(pivaloyloxymethyl)(9-[(R)-2-(phosphonomethoxy)ethyl]adenin oder einem pharmazeutisch akzeptablen Derivat davon ausgewählt ist, worin (2R,cis)-4-Amino-1-(2-hydroxymethyl-1,3-oxathiolan-5-yl)-pyrimidin-2-on und das zweite Therapeutikum im Gewichtsbereich von 40:1 bis 1:1 vorhanden sind, zur Herstellung eines Medikaments zu Behandlung einer HBV-Infektion.

11. Verwendung gemäß Anspruch 10, worin die Kombination wie in Anspruch 1 oder 2 beansprucht ist.

12. Verwendung gemäß Anspruch 10 oder 11 zur Behandlung einer HBV-Infektion, die gegen nukleosidische und/oder nicht-nukleosidische Inhibitoren der Vermehrung des Hepatitis B-Virus resistent ist.

13. Verwendung von (2R,cis)-4-Amino-1-(2-hydroxymethyl-1,3-oxathiolan-5-yl)-pyrimidin-2-on oder einem pharmazeutisch akzeptablen Derivat davon zur Herstellung eines Medikaments zur Verabreichung entweder gleichzeitig oder aufeinanderfolgend mit (9-[(R)-2-(Phosphonomethoxy)ethyl]adenin oder einem pharmazeutisch akzeptablen Derivat davon oder Bis(pivaloyloxymethyl)(9-[2-(phosphonomethoxy)ethyl]adenin oder einem pharmazeutisch akzeptablen Derivat davon, worin (2R,cis)-4-Amino-1-(2-hydroxymethyl-1,3-oxathiolan-5-yl)-pyrimidin-2-on und (9-[(R)-2-(Phosphonomethoxy)ethyl]adenin oder Bis(pivaloyloxymethyl)(9-[2-(phosphonomethoxy)ethyl]adenin im Gewichtsbereich von 40:1 bis 1:1 vorhanden sind, zur Behandlung einer HBV-Infektion.

14. Verwendung von (9-[(R)-2-(Phosphonomethoxy)ethyl]adenin oder einem pharmazeutisch akzeptablen Derivat davon oder Bis(pivaloyloxymethyl)-(9-[2-(phosphonomethoxy)ethyl]adenin oder einem pharmazeutisch akzeptablen Derivat davon zur Herstellung eines Medikaments zur Verabreichung entweder gleichzeitig oder aufeinanderfolgend mit (2R,cis)-4-Amino-1-(2-hydroxymethyl-1,3-oxathiolan-5-yl)-(1H)-pyrimidin-2-on oder einem pharmazeutisch akzeptablen Derivat davon, worin (9-[(R)-2-(Phosphonomethoxy)ethyl]adenin oder Bis(pivaloyloxymethyl) (9-[2-(phosphonomethoxy)ethyl]adenin und (2R,cis)-4-Amino-1-(2-hydroxymethyl-1,3-oxathiolan-5-yl)-pyrimidin-2-on im Gewichtsbereich von 1:40 bis 1:1 vorhanden sind, zur Behandlung einer HBV-Infektion.

15. Produkt, das (2R,cis)-4-Amino-1-(2-hydroxymethyl-1,3-oxathiolan-5-yl)-pyrimidin-2-on oder ein pharmazeutisch akzeptables Derivat davon umfaßt, als Zubereitung zur gleichzeitigen, getrennten oder aufeinanderfolgenden Verwendung zusammen mit Bis(pivaloyloxymethyl)(9-[2-(phosphonomethoxy)ethyl]adenin oder einem pharmazeutisch akzeptablen Derivat davon in einem Gewichtsverhältnis von 40:1 bis 1:1 in der Behandlung einer HBV-Infektion.

16. Produkt, das Bis(pivaloyloxymethyl)(9-[2-(phosphonomethoxy)ethyl]adenin oder ein pharmazeutisch akzeptables Derivat davon umfaßt, als Zubereitung zur gleichzeitigen, getrennten oder aufeinanderfolgenden Verwendung zusammen mit (2R,cis)-4-Amino-1-(2-hydroxymethyl-1,3-oxathiolan-5-yl)-pyrimidin-2-on oder einem pharmazeutisch akzeptablen Derivat davon in einem Gewichtsverhältnis von 1:40 bis 1:1 in der Behandlung einer HBV-Infektion.

17. Produkt, das (2R,cis)-4-Amino-1-(2-hydroxymethyl-1,3-oxathiolan-5-yl)-pyrimidin-2-on oder ein pharmazeutisch akzeptables Derivat davon und Bis(pivaloyloxymethyl)(9-[2-(phosphonomethoxy)ethyl]adenin oder ein pharmazeutisch akzeptables Derivat davon in einem Gewichtsverhältnis von 40:1 bis 1:1 als kombinierte Zubereitung zur gleichzeitigen, getrennten oder aufeinanderfolgenden Verwendung in der Behandlung einer HBV-Infektion umfaßt.

## Revendications

1. Combinaison comprenant la (2R,cis)-4-amino-1-(2-hydroxyméthyl-1,3-oxathiolan-5-yl)-pyrimidin-2-one ou un dérivé de celle-ci acceptable du point de vue pharmaceutique et un second agent thérapeutique, la bis(pivaloyloxyméthyl)(9-[(R)-2-(phosphonométhoxy)éthyl]adénine ou un dérivé de celle-ci acceptable du point de vue pharmaceutique, dans laquelle la (2R,cis)-4-amino-1-(2-hydroxyméthyl-1,3-oxathiolan-5-yl)-pyrimidin-2-one et le second agent thérapeutique sont présents dans la gamme de 40 :1 à 1 :1 en poids.

2. Combinaison selon la revendication 1, dans laquelle le rapport est compris dans la gamme de 25 :1 à 15 :1 en poids des composants actifs.

3. Combinaison selon les revendications 1 ou 2, utile en médecine.

4. Formulation pharmaceutique comprenant une combinaison selon l'une quelconque des revendications 1 à 3 en association avec un ou plusieurs supports acceptables du point de vue pharmaceutique pour celle-ci.

5. Formulation pharmaceutique utile dans le traitement de HBV (virus de l'hépatite B) comprenant la (2R,cis)-4-amino-1-(2-hydroxyméthyl-1,3-oxathiolan-5-yl)-pyrimidin-2-one ou un dérivé de celle-ci acceptable du point de vue pharmaceutique et un second agent thérapeutique sélectionné parmi la (9-[(R)-2-(phosphonométhoxy)éthyl]adénine ou un dérivé de celle-ci acceptable du point de vue pharmaceutique, et la bis(pivaloyloxyméthyl)(9-[(R)-2-(phosphonométhoxy)éthyl]adénine ou un dérivé de celle-ci acceptable du point de vue pharmaceutique, dans laquelle la (2R,cis)-4-amino-1-(2-hydroxyméthyl-1,3-oxathiolan-5-yl)-pyrimidin-2-one et le second agent thérapeutique sont présents dans la gamme de 40 :1 à 1 :1 en poids.

6. Formulation selon les revendications 4 ou 5 sous forme de dose unitaire.

7. Formulation selon l'une quelconque des revendications 4 à 6 convenable pour l'administration orale.

8. Formulation selon l'une quelconque des revendications 5 à 7 comprenant entre 25 et 150 mg de (2R,cis)-4-amino-1-(2-hydroxyméthyl-1,3-oxathiolan-5-yl)-pyrimidin-2-one et entre 5 et 60 mg de bis(pivaloyloxyméthyl)(9-[(R)-2-(phosphonométhoxy)éthyl]adénine.

9. Formulation selon la revendication 8, comprenant 100 mg de (2R,cis)-4-amino-1-(2-hydroxyméthyl-1,3-oxathiolan-5-yl)-pyrimidin-2-one et 10 mg de bis(pivaloyloxyméthyl)(9-[(R)-2-(phosphonométhoxy)éthyl]adénine.

10. Utilisation d'une combinaison comprenant la (2R,cis)-4-amino-1-(2-hydroxyméthyl-1,3-oxathiolan-5-yl)-pyrimidin-2-one ou un dérivé de celle-ci acceptable du point de vue pharmaceutique et un second agent thérapeutique sélectionné parmi la (9-[(R)-2-(phosphonométhoxy)éthyl]adénine ou un dérivé de celle-ci acceptable du point de vue pharmaceutique et la bis(pivaloyloxyméthyl)(9-[(R)-2-(phosphonométhoxy)éthyl]adénine ou un dérivé de celle-ci acceptable du point de vue pharmaceutique, dans laquelle la (2R,cis)-4-amino-1-(2-hydroxyméthyl-1,3-oxathiolan-5-yl)-pyrimidin-2-one et le second agent thérapeutique sont présents dans la gamme de 40 :1 à 1 :1 en poids, pour la fabrication d'un médicament pour le traitement d'une infection par HBV.

11. Utilisation selon la revendication 10, dans laquelle la combinaison est selon les revendications 1 ou 2.

12. Utilisation selon les revendications 10 ou 11 pour le traitement d'une infection par HBV résistant à des inhibiteurs de type nucléoside et/ou non nucléoside de la réplication du virus de l'hépatite B.

13. Utilisation de (2R,cis)-4-amino-1-(2-hydroxyméthyl-1,3-oxathiolan-5-yl)-pyrimidin-2-one ou d'un dérivé de celle-ci acceptable du point de vue pharmaceutique pour la fabrication d'un médicament à administrer soit simultanément, soit successivement avec la (9-[(R)-2-(phosphonométhoxy)éthyl]adénine ou un dérivé de celle-ci acceptable du point de vue pharmaceutique ou la bis(pivaloyloxyméthyl)(9-[(R)-2-(phosphonométhoxy)-éthyl]adénine ou un dérivé de celle-ci acceptable du point de vue pharmaceutique, où la (2R,cis)-4-amino-1-(2-hydroxyméthyl-1,3-oxathiolan-5-yl)-pyrimidin-2-one et la (9-[(R)-2-(phosphonométhoxy)éthyl]adénine ou la bis(pivaloyloxyméthyl)(9-[2-(phosphonométhoxy)éthyl]adénine sont présentes dans la gamme de 40 :1 à 1 :1 en poids, pour le traitement d'une infection par HBV.

14. Utilisation de (9-[(R)-2-(phosphonométhoxy)-éthyl]adénine ou d'un dérivé de celle-ci acceptable du point de vue pharmaceutique ou de la bis(pivaloyloxyméthyl)(9-[2-(phosphonométhoxy)éthyl]adénine ou d'un dérivé de celle-ci acceptable du point de vue pharmaceutique, pour la fabrication d'un médicament à administrer soit simultanément, soit successivement avec la (2R,cis)-4-amino-1-(2-hydroxyméthyl-1,3-oxathiolan-5-yl)-(1H)-pyrimidin-2-one ou un dérivé de celle-ci acceptable du point de vue pharmaceutique, où la (9-[2-(phosphonométhoxy)éthyl]adénine ou la bis(pivaloyloxyméthyl)(9-[2-(phosphonométhoxy)-éthyl]adénine et la (2R,cis)-4-amino-1-(2-hydroxyméthyl-1,3-oxathiolan-5-yl)-pyrimidin-2-one sont présentes dans la gamme de 1 :40 à 1 :1 en poids pour le traitement d'une infection par HBV.

15. Produit comprenant la (2R,cis)-4-amino-1-(2-hydroxyméthyl-1,3-oxathiolan-5-yl)-pyrimidin-2-one ou un dérivé de celle-ci acceptable du point de vue pharmaceutique en tant que préparation pour l'utilisation simultanée, séparée ou successive avec la bis(pivaloyloxyméthyl)(9-[2-(phosphonométhoxy)éthyl]adénine ou un dérivé de celle-ci acceptable du point de vue pharmaceutique selon un rapport compris dans la gamme de 40 :1 à 1 :1 en poids, dans le traitement d'une infection par HBV.

16. Produit comprenant la bis(pivaloyloxyméthyl)(9-[2-(phosphonométhoxy)éthyl]adénine ou un dérivé de celle-ci acceptable du point de vue pharmaceutique en tant que préparation pour l'utilisation simultanée, séparée ou successive avec la (2R,cis)-4-amino-1-(2-hydroxyméthyl-1,3-oxathiolan-5-yl)-pyrimidin-2-one ou un dérivé de celle-ci acceptable du point de vue pharmaceutique selon un rapport compris dans la gamme de 1 :40 à 1 :1 en poids, dans le traitement d'une infection par HBV.

17. Produit comprenant la (2R,cis)-4-amino-1-(2-hydroxyméthyl-1,3-oxathiolan-5-yl)-pyrimidin-2-one ou un dérivé de celle-ci acceptable du point de vue pharmaceutique et la bis(pivaloyloxyméthyl)(9-[2-(phosphonométhoxy)éthyl]adénine ou un dérivé de celle-ci acceptable du point de vue pharmaceutique selon un rapport compris dans la gamme de 40 :1 à 1 :1 en poids, en tant que préparation combinée pour l'utilisation simultanée, séparée ou successive dans le traitement d'une infection par HBV.

## FIG. 1

### COMBINATION ISOBOLOGRAM
### LAMIVUDINE VS ADEFOVIR

| NET SUM | -3.102370207 | SE | 1.29895894 |
| t | -2.388351257 | | |
| AV DEV | -0.141016828 | | |
| P (DEV) | 0.013449974 | | |